# FASCICULE DE BREVET EUROPEEN

(11) **EP 1 217 957 B1**
(45) Date de publication et mention de la délivrance du brevet: **10.03.2004**
(21) Numéro de dépôt: 00960934.8
(22) Date de dépôt: 02.10.2000
(51) Int. Cl.: A61B 17/225, G10K 15/06

(54) **APPAREIL LITHOTRITEUR**
LITHOTRIPSIEVORRICHTUNG
LITHOTRIPTER APPARATUS

(30) Priorité: 01.10.1999 FR 9912431
(43) Date de publication de la demande: 03.07.2002
(73) Titulaire: Internova International Innovation Company B.V., 3012 CA Rotterdam (NL)
(72) Inventeur: SANGOUARD, Patrick, F-94350 Villiers sur Marne (FR)
(74) Mandataire: Nithardt, Roland
(86) Numéro de dépôt international: PCT/IB2000/001400
(87) Numéro de publication internationale: WO 2001/024712

(56) Documents cités:
- EP-A- 0 349 915
- EP-A- 0 449 138
- FR-A- 2 593 383
- US-A- 4 608 983
- US-A- 5 583 901

## Description

### Domaine technique

La présente invention concerne un appareil lithotriteur comportant un bâti, une table de traitement pour supporter un patient pendant un traitement de fractionnement d'une lithiase, un dispositif pour générer des ondes de choc au moyen d'un arc électrique, des moyens pour focaliser ces ondes de choc sur ladite lithiase, et un dispositif de visualisation en direct de la zone de traitement, ledit dispositif pour générer des ondes de choc comportant deux électrodes écartées l'une de l'autre et connectées à un circuit générateur d'énergie de haute tension, et les moyens pour focaliser ces ondes de choc comportant un réflecteur semi-éllipsoidal, les extrémités desdites électrodes étant disposées sensiblement à l'un des foyers du réflecteur, l'autre foyer dudit réflecteur étant centré sur ladite lithiase, lesdites électrodes étant équipées de moyens de compensation de leur usure.

### Technique antérieure

Un lithotriteur est un appareil conçu pour assurer le fractionnement ou la destruction des calculs rénaux ou lithiases, par exemple au moyen d'ondes de choc générées par un arc électrique et focalisées en des points situés à l'intérieur des calculs localisés dans les reins. Les ondes de choc sont générées par des arcs électriques qui sont produits à l'un des foyers, appelé foyer émetteur, d'un réflecteur semi-éllipsoidal métallique qui forme une cuve. Cette cuve est remplie d'eau salée pendant l'intervention et elle est recouverte d'une membrane souple et étanche qui est appliquée directement sur la peau du patient pour assurer la continuité du milieu conducteur des ondes de choc. Le réflecteur est positionné de telle manière que l'autre foyer, appelé foyer récepteur, se situe à l'intérieur de la lithiase à fractionner ou a détruire.

L'efficacité d'un tel appareil et la fiabilité du traitement reposent principalement sur la précision du positionnement du foyer récepteur sur la cible. A cet effet, deux moyens de contrôle sont indispensables. L'un concerne le positionnement précis par rapport au foyer émetteur des électrodes qui génèrent l'arc électrique et l'autre concerne le positionnement précis du foyer récepteur par rapport à la lithiase, cette dernière devant être visualisée en direct pour permettre ce positionnement précis.

On connaît divers appareils agencés pour effectuer ce traitement. Cependant, ils présentent tous des défauts et des inconvénients qui rendent soit leur construction compliquée et coûteuse, soit leur utilisation peu précise et par conséquent le traitement du patient aléatoire et peu efficace. L'un de ces inconvénients trouve son origine dans le manque de précision de la position du générateur d'ondes de choc, idéalement placé au foyer émetteur de la cuve, notamment en raison de l'usure des électrodes qui composent ce générateur. L'écartement des extrémités opposées des électrodes par rapport à ce foyer engendre des imprécisions dans les tirs et est à l'origine de certains manques d'efficacité constatés. Un autre inconvénient trouve son origine dans l'imprécision de la localisation de la lithiase. La détermination précise de cette position est essentielle afin de permettre au personnel soignant de faire coïncider le foyer récepteur du réflecteur avec ladite lithiase et ainsi d'assurer l'efficacité de l'appareil.

En particulier, la publication FR 2 593 383, correspondant au préambule de la première revendication a pour objet un appareil générateur d'ondes de choc de fréquence pour la destruction de cibles telles que des calculs rénaux. Cet appareil comporte deux électrodes mobiles en translation axiale et placées transversalement par rapport à l'axe de révolution du réflecteur. De par cette conception, d'une part, la disposition des électrodes masque en partie le champ de génération des ondes de choc et, d'autre part, le déplacement uniquement axial des électrodes aboutit à une usure irrégulière de ces électrodes et donc à une diminution de la précision du positionnement sur le foyer émetteur.

Ce dernier inconvénient se retrouve également dans l'appareil décrit par brevet US 4 608 983 étant donné que, de manière similaire, les électrodes ne sont mobiles qu'en translation axiale.

Certains autres appareils connus comportent des moyens permettant de déplacer le patient par rapport à l'appareil pour amener la lithiase et le foyer récepteur en coïncidence ou pour amener le foyer récepteur mécaniquement lié à l'appareil en coïncidence avec la lithiase. Dans les deux cas, les constructions sont lourdes et encombrantes. En outre, tous les composants, et en particulier les moyens de visualisation, sont dédiés, ce qui renchérit considérablement les appareils.

### Exposé de l'invention

L'appareil selon l'invention permet de pallier ces différents inconvénients et d'assurer avec efficacité le fractionnement des lithiases en leur appliquant des ondes de choc d'une puissance parfaitement adaptée et d'une manière parfaitement ciblée, ceci afin d'éviter des lésions des tissus environnants. Ceci est dû particulièrement au fait que le système de compensation de l'usure des électrodes tel que réalisé permet d'éviter une usure en biais des électrodes qui engendre un décentrage du générateur d'ondes de choc par rapport au foyer émetteur du réflecteur. En outre, cet appareil est peu encombrant par rapport aux appareils connus et permet des interventions faciles, rapides et efficaces, les tirs étant d'une très grande précision. De plus les équipements annexes, tels que les dispositifs de visualisation, ne sont pas dédiés et peuvent être utilisés pour d'autres interventions.

Ce but est atteint par l'appareil lithotriteur selon l'invention, comportant des moyens pour compenser l'usure des électrodes comportent au moins un mécanisme de compensation agencé pour déplacer axialement les deux électrodes, indépendamment l'une de l'autre, selon un axe correspondant à l'axe de symétrie du réflecteur et pour faire tourner au moins l'une desdites électrodes autour de son axe longitudinal. De ce fait, les extrémités desdites électrodes s'usent de façon régulière et peuvent toujours être positionnées de façon précise pour que le générateur des ondes de choc qu'elles constituent soit centré sur le foyer émetteur du réflecteur formant la cuve, et ceci quel que soit le degré d'usure de ces électrodes. Il en résulte que l'une des deux conditions de base énoncées ci-dessus, à savoir que les ondes de choc soient bien ciblées sur les lithiases à traiter, est remplie.

De façon particulièrement avantageuse, l'une des électrodes est disposée selon l'axe du réflecteur et logée à l'intérieur d'un tube qui est couplé à un premier mécanisme agencé pour déplacer axialement ledit tube avec ladite électrode qu'il contient et pour entraîner ce tube en rotation selon son axe longitudinal. L'autre électrode comprend deux parties reliées entre elles par une barrette, l'une de ces parties étant disposée selon l'axe du réflecteur et l'autre partie étant logée à l'intérieur d'un tube qui est couplé à un deuxième mécanisme agencé pour déplacer axialement ledit tube avec l'électrode qu'il contient.

De préférence, l'appareil comporte un calculateur agencé pour déterminer respectivement les coordonnées spatiales de la lithiase à traiter et du réflecteur, des moyens pour émettre des signaux de commande en fonction des valeurs déterminées desdites coordonnées spatiales, et des moyens pour déplacer ledit réflecteur en fonction desdits signaux de manière que le foyer récepteur soit positionné sur ladite lithiase. Cette manière de procéder permet de garantir une grande précision dans le positionnement de la lithiase et d'obtenir une souplesse d'utilisation inégalée.

De façon avantageuse, les moyens pour déplacer ledit réflecteur comportent un boîtier qui porte la cuve constituant ledit réflecteur, un chariot sur lequel est monté ledit boîtier, des coulisses sensiblement horizontales et croisées pour permettre un déplacement dudit chariot selon deux directions orthogonales, des coulisses sensiblement verticales pour permettre son déplacement selon une troisième direction perpendiculaire aux deux autres directions, ainsi que des organes d'actionnement de ce chariot selon ces trois directions. De ce fait, on déplace simplement le générateur d'ondes de choc sans être obligé de déplacer le patient ou l'appareil tout entier.

Selon un premier mode de réalisation avantageux, ledit dispositif de visualisation en direct comporte un équipement de radioscopie comprenant un générateur de rayons X et des moyens de réception desdits rayons respectivement montés aux deux extrémités d'un bras en forme d'arc de cercle pivotant dans son plan autour de son centre, un écran d'affichage des images radioscopiques et des moyens pour communiquer audit calculateur les données visualisées en vue de la détermination des coordonnées relatives du foyer émetteur et de la lithiase.

Selon un deuxième mode de réalisation, ledit dispositif de visualisation en direct comporte un équipement d'échographie ainsi qu'au moins deux caméras de prises de vues décalées l'une par rapport à l'autre, un écran d'affichage des images échographiques et des moyens pour communiquer audit calculateur les données visualisées en vue de la détermination des coordonnées relatives du foyer émetteur et de la lithiase.

Dans ce cas, l'une des caméras est avantageusement disposée au bas de l'appareil, sous la zone de traitement, pour pouvoir fournir une image sensiblement perpendiculairement par rapport à cette zone, et l'autre caméra est décalée sur le côté pour pouvoir fournir une image selon une direction oblique de cette zone.

### Description sommaire des dessins

La présente invention sera mieux comprise en référence à la description ci-dessous d'une forme de réalisation préférée d'un appareil lithotriteur selon l'invention et en référence aux dessins, donnés à titre d'exemples non limitatifs, dans lesquels:
la figure 1 représente une vue d'ensemble en perspective de l'appareil selon l'invention,
la figure 2 est une vue schématique illustrant le principe de fonctionnement de l'appareil selon l'invention,
la figure 3 représente schématiquement une vue en coupe axiale du réflecteur et des électrodes de l'appareil lithotriteur selon l'invention,
la figure 4 représente une vue d'une forme de réalisation particulière du dispositif de visualisation lié à l'appareil selon l'invention.

### Manière(s) de réaliser l'invention

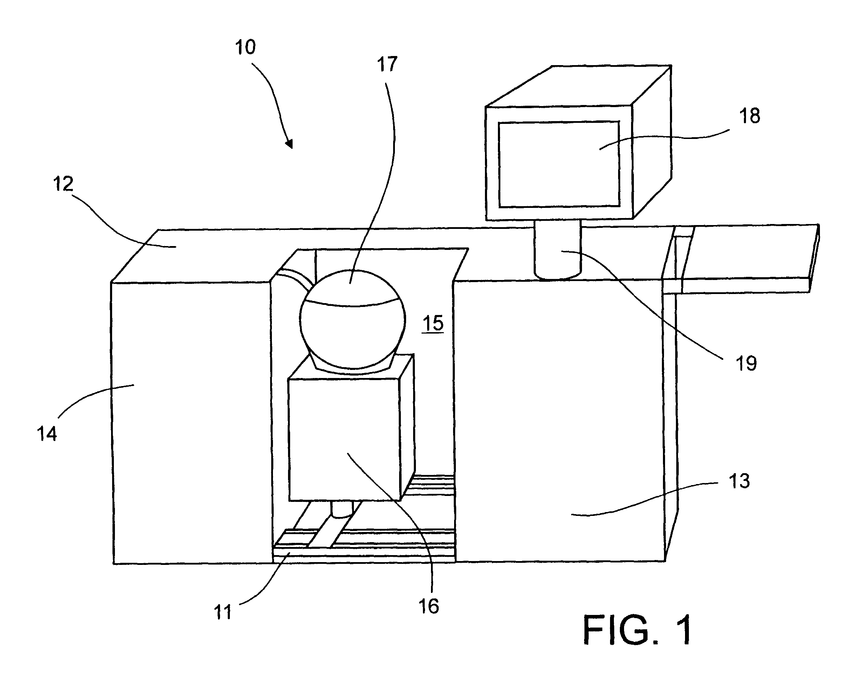

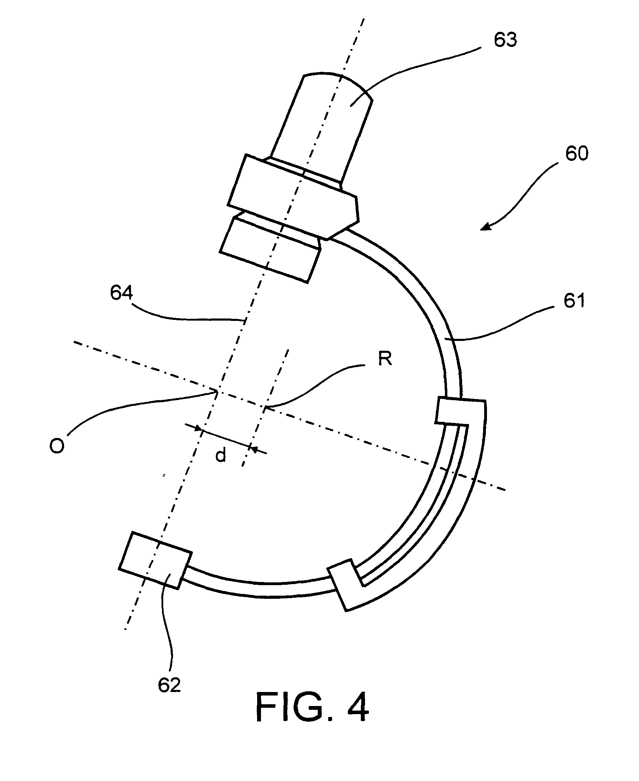

En référence à la figure 1, l'appareil lithotriteur 10 tel que représenté comporte essentiellement un bâti 11 surmonté d'une table de traitement 12 destinée à supporter un patient à soigner. Le bâti 11 se présente sous la forme d'un coffre dont la partie droite 13 contient essentiellement des équipements électriques et électroniques, dont la partie gauche 14 contient essentiellement des équipements mécaniques et dont la partie centrale 15 contient principalement un chariot mobile 16 qui porte un réflecteur semi-ellipsoïdal 17 et un dispositif pour générer les ondes de choc destinées à provoquer la dislocation des lithiases. Un écran 18 porté par un support orientable 19 est monté sur le bâti 11. Un dispositif de visualisation en direct de la zone de traitement, dont une forme de réalisation sera décrite en référence à la figure 4, est associé de préférence de manière indépendante à l'appareil et à l'écran 18.

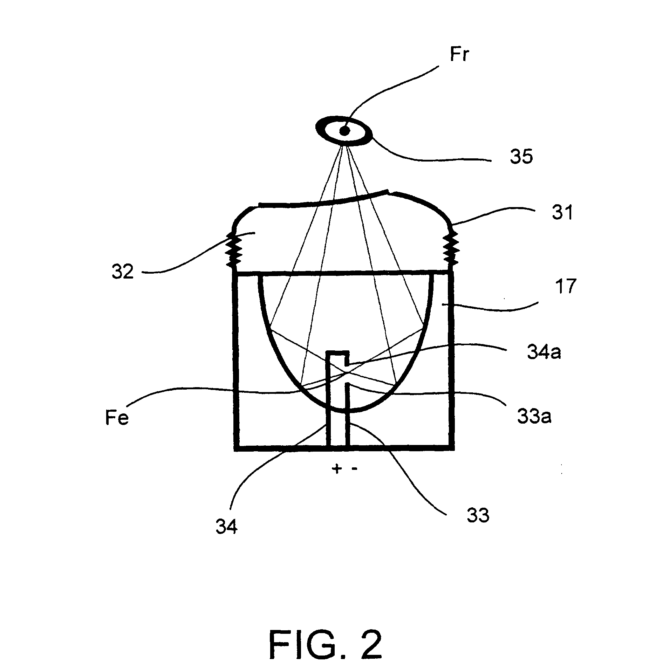

La figure 2 représente schématiquement le réflecteur 17 surmonté d'une membrane 31 et contenant un liquide acoustique transmetteur 32, agencé pour assurer la continuité du milieu transmetteur des ondes de choc lorsque la membrane est appliquée contre le corps du patient. Le réflecteur semi-ellipsoïdal 17 en forme de cuve contient deux électrodes 33 et 34 dont les extrémités 33a et 34a, disposées en regard l'une de l'autre, sont positionnées axialement sensiblement au foyer émetteur Fₑ de l'ellipsoïde. La formation d'un arc électrique entre les deux électrodes, consécutif à la décharge d'un condensateur dans ces électrodes provoque une onde de pression qui est réfléchie par les parois de la cuve.

Comme le montre la figure, les ondes de choc générées par les électrodes 33 et 34 sont focalisées sur une lithiase 35 présente dans le rein d'un patient et positionnée de sorte à coïncider avec le foyer récepteur Fᵣ du réflecteur. Les électrodes 33 et 34 constituent un générateur électro-hydraulique d'ondes de pression à haut rendement.

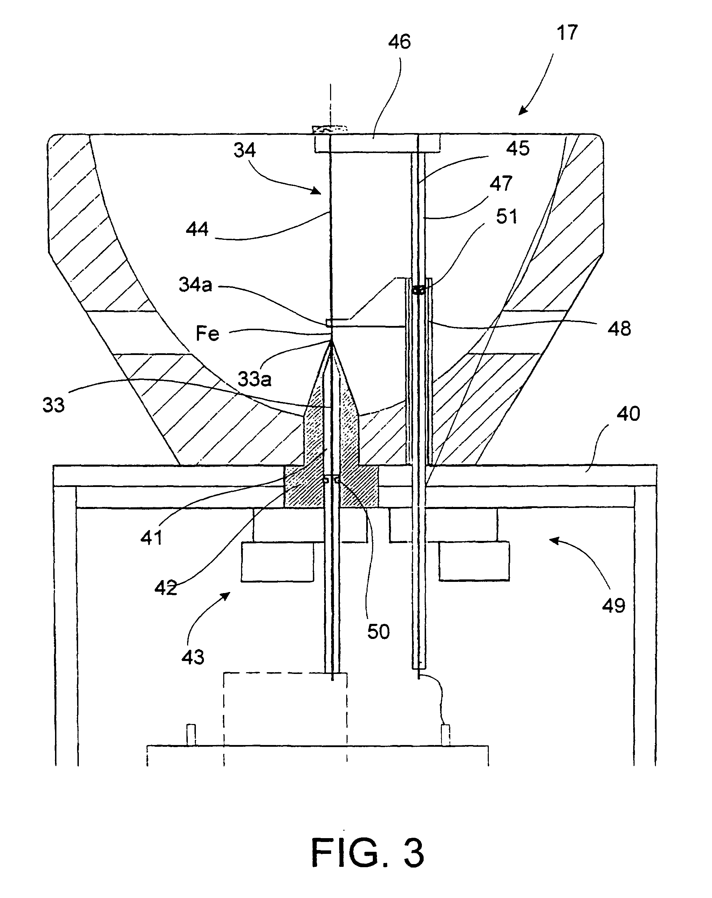

La figure 3 est une vue en coupe axiale de la cuve qui constitue le réflecteur semi-ellipsoïdal 17 et des électrodes qui constituent le générateur électro-hydraulique d'ondes de pression. La cuve est fixée sur un boîtier 40 et contient les deux électrodes 33 et 34 disposées de manière que leurs extrémités respectives 33a et 34a, terminées en pointe, se situent sur l'axe de symétrie de cette cuve, de part et d'autre du foyer émetteur Fₑ du réflecteur. L'électrode 33 traverse un tube porte-électrode 41 en matière plastique logé à l'intérieur d'un embout fixe 42 qui est monté axialement au fond de la cuve. Ce tube porte-électrode 41 est couplé à un mécanisme d'entraînement comprenant un moto-réducteur 43 qui a pour première fonction d'assurer un déplacement axial de l'électrode 33 afin de compenser son usure et de ramener constamment son extrémité 33a dans sa position axiale à proximité du foyer émetteur Fₑ, et pour deuxième fonction d'engendrer une rotation de l'électrode 33 autour de son axe afin d'éviter une usure irrégulière en biais qui aurait pour effet préjudiciable un décalage de l'extrémité 33a par rapport au foyer émetteur Fₑ.

L'électrode 34 est constituée de deux parties 44 et 45 parallèles entre elles et reliées par une barrette 46. La partie 45 est logée dans un tube porte-électrode 47 qui traverse le fond de la cuve dans un embout 48 approprié. Ce tube porte-électrode 47 est couplé à un mécanisme d'entraînement comprenant un moto-réducteur 49 qui a pour fonctions d'assurer un déplacement axial de l'électrode 34 pour compenser son usure et de ramener constamment son extrémité 34a dans sa position axiale à proximité du foyer émetteur Fₑ.

L'embout 42 contient un joint torique 50 qui assure l'étanchéité du tube porte-électrode 41. De façon similaire, l'embout 48 contient un joint torique 51 qui assure l'étanchéité du tube porte-électrode 47.

La figure 4 représente une partie de l'appareil qui constitue le dispositif de visualisation en direct 60 de la zone de traitement. Ce dispositif est de préférence indépendant de l'appareil, mais peut également être monté à demeure. Il s'agit, dans l'exemple représenté, d'un bras de radioscopie 61 en forme d'arceau, appelé bras en C, qui porte à son extrémité inférieure un générateur 62 de rayons X et à son extrémité supérieure des moyens de réception desdits rayons, tels qu'un amplificateur de brillance 63. Le bras en C est circulaire et pivote dans son plan autour de son centre de pivotement R qui est décalé par rapport à l'axe 64 défini par le générateur 62 et l'amplificateur 63. Le décalage d existant entre R et le point d'intersection O de l'axe 64 avec l'axe perpendiculaire passant par le centre de pivotement R est de l'ordre de 40 à 80 mm.

Dans une autre forme de réalisation, ce dispositif de visualisation par rayons X peut être remplacé par un dispositif de repérage des lithiases par échographie qui comprend, d'une manière connue en soi, une sonde d'échographie et l'écran 18 qui affiche les images captées.

Au cours de l'opération, on procède à deux prises de vues successives. L'une des prises de vues se fait perpendiculairement à la table 12 servant à supporter le patient et l'autre se fait obliquement par rapport à la table. Les coordonnées trouvées et affichées sur l'écran sont reportées sur un calculateur qui détermine avec précision les coordonnées du générateur d'ondes de choc. Cette démarche constitue une opération de calibrage de l'appareil par triangulation. Elle est rendue possible par le fait que le bras en C peut pivoter dans son plan autour de son centre de pivotement qui est disposé au centre géométrique de l'arceau de forme circulaire. Après la mise en place du patient sur la table d'intervention, on procède de la même manière pour déterminer la position de la lithiase. On effectue une première radioscopie de la zone de traitement avec le bras de radioscopie en position verticale et une deuxième radioscopie de cette zone avec le bras en position oblique, l'inclinaison étant par exemple de l'ordre de 20 degrés par rapport à la verticale. Les coordonnées de la lithiase sur les deux images sont introduites dans un calculateur. Ce dernier détermine alors avec précision la position du générateur d'ondes de choc pour que le foyer émetteur Fₑ soit positionné de telle manière que le foyer récepteur Fᵣ corresponde à la lithiase.

Une démarche similaire peut être effectuée en remplaçant la radiographie par l'échographie. Dans cette réalisation l'appareil comporte deux caméras de prises de vues, dites caméras de repérage, dont l'une est disposée respectivement au bas de l'appareil, sous la zone de traitement, pour pouvoir fournir une image sensiblement perpendiculairement par rapport à cette zone et l'autre est décalée sur le côté pour pouvoir fournir une image selon une direction oblique de cette zone. Lorsque l'opérateur fait une échographie du patient à l'aide d'une sonde échographique, les caméras de repérage enregistrent la position de la sonde dans l'espace de l'appareil. Un calculateur détermine les coordonnées spatiales de cette position, ce qui permet ensuite de déduire la position de la lithiase et de définir avec précision la position du générateur d'ondes de choc pour que le foyer émetteur Fₑ soit positionné de telle manière que le foyer récepteur Fᵣ corresponde à la lithiase.

Après la détermination de ces coordonnées, il convient d'amener le foyer émetteur Fₑ dans sa bonne position. A cet effet, l'appareil comporte des moyens pour déplacer le réflecteur 17, ou plus exactement le boîtier 40 qui porte la cuve constituant le réflecteur, à l'emplacement voulu. Ces moyens comportent un chariot sur lequel est monté ledit boîtier, des coulisses sensiblement horizontales et croisées pour permettre un déplacement de ce chariot selon deux directions orthogonales, des coulisses sensiblement verticales pour permettre son déplacement selon une troisième direction perpendiculaire aux deux autres directions, ainsi que des organes d'actionnement de ce chariot selon ces trois directions. Ces moyens sont commandés par des signaux générés par le calculateur, ou un générateur de signaux couplé à ce calculateur, et transmis de préférence à des moteurs pas à pas couplés à des organes mécaniques qui assurent les déplacements du chariot.

## Revendications

1. Appareil lithotriteur comportant un bâti (11), une table de traitement (12) pour supporter un patient pendant un traitement de fractionnement d'une lithiase (35), un dispositif pour générer des ondes de choc au moyen d'un arc électrique, des moyens pour focaliser ces ondes de choc sur ladite lithiase, et un dispositif de visualisation en direct (60) de la zone de traitement, ledit dispositif pour générer des ondes de choc comportant deux électrodes (33, 34) écartées l'une de l'autre et connectées à un circuit générateur d'énergie de haute tension, et les moyens pour focaliser ces ondes de choc comportant un réflecteur semi-ellipsoïdal (17), les extrémités (33a, 34a) de ces électrodes étant disposées sensiblement à l'un (Fₑ) des foyers du réflecteur (17), l'autre foyer (Fᵣ) du réflecteur étant centré sur ladite lithiase, lesdites électrodes étant équipées de moyens pour compenser leur usure, lesdits moyens comportent au moins un mécanisme de compensation agencé pour déplacer axialement les deux électrodes, indépendamment l'une de l'autre, **caractérisé en ce que** ledit mécanisme de compensation est agencé pour déplacer ces deux électrodes selon un axe correspondant à l'axe de symétrie du réflecteur (17) et pour faire tourner au moins l'une desdites électrodes autour de son axe longitudinal.

2. Appareil selon la revendication 1, **caractérisé en ce que** l'une (33) des électrodes est disposée selon l'axe du réflecteur (17) et logée à l'intérieur d'un tube (41) qui est couplé à un premier mécanisme agencé pour déplacer axialement ledit tube avec l'électrode qu'il contient et pour entraîner ce tube en rotation selon son axe longitudinal.

3. Appareil selon la revendication 1, **caractérisé en ce que** l'autre électrode (34) comprend deux parties (44, 45) reliées entre elles par une barrette (46), l'une (44) de ces parties étant disposée selon l'axe du réflecteur (17) et l'autre partie (45) étant logée à l'intérieur d'un tube (47) qui est couplé à un deuxième mécanisme agencé pour déplacer axialement ledit tube avec l'électrode qu'il contient.

4. Appareil selon la revendication 1, **caractérisé en ce qu'**il comporte un calculateur agencé pour déterminer les coordonnées spatiales de la lithiase à traiter (35) et du réflecteur (17), des moyens pour émettre des signaux de commande en fonction des valeurs déterminées desdites coordonnées spatiales, et des moyens pour déplacer ledit réflecteur en fonction desdits signaux de manière que le foyer récepteur (Fᵣ) soit positionné sur ladite lithiase.

5. Appareil selon la revendication 4, **caractérisé en ce que** les moyens pour déplacer le réflecteur (17) comportent un boîtier (40) qui porte la cuve constituant ledit réflecteur, un chariot sur lequel est monté ledit boîtier, des coulisses sensiblement horizontales et croisées pour permettre un déplacement de ce chariot selon deux directions orthogonales, des coulisses sensiblement verticales pour permettre son déplacement selon une troisième direction perpendiculaire aux deux autres directions, ainsi que des organes d'actionnement de ce chariot selon ces trois directions.

6. Appareil selon la revendication 1, **caractérisé en ce que** le dispositif de visualisation en direct (60) comporte un équipement de radioscopie comprenant un générateur de rayons X (62) et des moyens de réception (63) desdits rayons respectivement montés aux deux extrémités d'un bras en forme d'arc de cercle (61) pivotant dans son plan autour de son centre, un écran d'affichage (18) des images radioscopiques et des moyens pour communiquer les données visualisées audit calculateur en vue de la détermination des coordonnées relatives du foyer émetteur (Fₑ) et de la lithiase.

7. Appareil selon la revendication 1, **caractérisé en ce que** le dispositif de visualisation en direct (60) comporte un équipement d'échographie ainsi qu'au moins deux caméras de prises de vues décalées l'une par rapport à l'autre, un écran d'affichage des images échographiques et des moyens pour communiquer les données visualisées audit calculateur en vue de la détermination des coordonnées relatives du foyer émetteur (Fₑ) et de la lithiase.

8. Appareil selon la revendication 7, **caractérisé en ce que** l'une des caméras est disposée au bas de l'appareil, sous la zone de traitement, pour pouvoir fournir une image sensiblement perpendiculairement par rapport à cette zone et l'autre caméra est décalée sur le côté pour pouvoir fournir une image selon une direction oblique de cette zone.

## Patentansprüche

1. Lithotriptor, bestehend aus einem Gestell (11), aus einem Behandlungstisch (12) zum Aufbahren eines Patienten während einer Behandlung von Lithiasis (35) mittels Frakionierung, aus einer Vorrichtung zur Stosswellenübertragung mittels eines elektrischen Lichtbogens, aus Einrichtungen zur Fokussierung dieser Stosswellen auf die betreffende Lithiasis, aus einem Bildschirmgerät (60) zur direkten Visualisierung der Behandlungszone, die betreffende Vorrichtung zur Stosswellenübertragung aus zwei von eineinander entfernten und an einen Starkstromkreisgenerator angeschlossenen Elektroden (33, 34) bestehend, die Einrichtungen zur Fokussierung dieser Stosswellen einen semi-ellipsoid-förmigen Reflektor (17) enthaltend, wobei die äusseren Enden (33a, 34a) der beiden Elektroden sich an einen der Brennpunkte (Fₑ) des Reflektors (17) befinden, und der andere Brennpunkt (Fᵣ) des Reflektors auf die betreffende Lithiasis zentriert ist, die betreffenden Elektroden mit Einrichtungen ausgestattet, die ihrer Abnutzung entgegenwirken und dafür aus mindestens einem Ausgleichsmechanismus bestehen, entsprechend angeordnet sind, um die beiden Elektroden, unabhängig voneinander, axial zu versetzen, **dadurch gekennzeichnet, dass** der betreffende Ausgleichsmechanismus so angelegt ist, um diese beiden Elektroden zu verschieben, auf einer Achse, die der Symmetrieachse des Reflektors (17) entspricht und mindestens eine der beiden Elektroden zum Rotieren um ihre Längsachse bringt.

2. Lithotriptor gemäss Patentanspruch 1 **dadurch gekennzeichnet, dass** eine (33) der beiden Elektroden der Achse des Reflektors (17) entspricht, inmitten eines Rohrs (41), das mit einem ersten Mechanismus verbunden ist, der so angelegt ist, um das betreffende Rohr mitsamt der enthaltenden Elektrode axial zu versetzen und das betreffende Rohr zum Rotieren um die Längsachse zu bringen.

3. Lithotriptor gemäss Patentanspruch 1 **dadurch gekennzeichnet, dass** die andere Elektrode (34) aus zwei durch einen Stab (46) miteinander verbundenen Teilen (44, 45) besteht, wobei das eine Teil (44) der Achse des Reflektors (17) entspricht und das andere Teil (45) im Inneren des Rohrs (47), das Rohr an einen zweiten Mechanismus gebunden ist, der das betreffende Rohr mitsamt der enthaltenden Elektrode axial versetzt.

4. Lithotriptor gemäss Patentanspruch 1 **dadurch gekennzeichnet, dass** er einen Rechner enthält zur Ermittlung der Raumkoordinaten der zu behandelnden Lithiasis (35) sowie des Reflektors (17), Einrichtungen zum Senden von Befehlssignalen im Zusammenhang mit den ermittelten Werten der Raumkoordinaten, sowie Einrichtungen um den betreffenden Reflektor in Abhängigkeit von den entsprechenden Signalen auf die Weise zu verschieben, dass der Fokus-Empfänger (Fᵣ) auf der betreffenden Lithiasis positionniert ist.

5. Lithotriptor gemäss Patentanspruch 4 **dadurch gekennzeichnet, dass** die Einrichtungen zum Versetzen des Reflektors (17) ein Gehäuse (40) enthalten, das den Reflektor darstellenden Behälter trägt sowie einen Wagen, auf dem das betreffende Gehäuse montiert ist, horizontal und gekreuzt angelegte Schienen um diesem Wagen eine Verlagerung in zwei orthogonale Richtungen zu erlauben, vertikal angelegte Schienen um seine Versetzung in eine dritte, senkrecht zu den zwei anderen Richtungen verlaufende Richtung zu ermöglichen, sowie Antriebsteile für diesen Wagen in die drei Richtungen.

6. Lithotriptor gemäss Patentanspruch 1 **dadurch gekennzeichnet, dass** das Bildschirmgerät zur Direktübertragung (60) eine Radioskopieaustattung mitsamt einem Röntgenstrahlgenerator (62) enthält, sowie Vorrichtungen (63) zum Empfang der betreffenden Strahlen, die respektive an den beiden äusseren Enden eines Arms in Form eines Kreisbogens (61) montiert sind, der sich in seiner Ebene um seinen Zentrum dreht, einem Bildschirm (18) zum Aufzeigen der Radioskopiebilder sowie Einrichtungen zur Übermittlung der visualisierten Daten an den Rechner im Hinblick auf die Erstellung der Koordinaten im Zusammenhang mit dem Fokus-Sender (Fₑ) und der Lithiasis.

7. Lithotriptor gemäss Patentanspruch 1 **dadurch gekennzeichnet, dass** das Bildschirmgerät zur Direktübertragung (60) eine Echografieausstattung enthält, sowie zumindest zwei voneinander entfernte Kameras zur Bildaufnahme, einen Bildschirm zum Aufblenden der Echografiebilder sowie Vorrichtungen zur Übermittlung der visualisierten Daten an den Rechner im Hinblick auf die Erstellung der Koordinaten im Zusammenhang mit dem Fokus-Sender (Fₑ) und der Lithiasis.

8. Lithotriptor gemäss Patentanspruch 7, **dadurch gekennzeichnet, dass** eine der beiden Kameras unten am Apparat installiert ist, unterhalb der Behandlungszone, um von dieser Zone ein senkrechtes Bild zu liefern und die andere Kamera auf der Seite montiert ist, um ein schräges Bild der betreffenden Zone zu liefern.

## Claims

1. A lithotriptor apparatus comprising a frame (11), a treatment table (12) for supporting a patient during the process of fragmenting a lithiasis (35), a device for generating shock waves by means of an electrical arc, means for focusing these shock waves on said lithiasis, and a device (60) for live viewing of the treatment zone, said device for generating shock waves comprising two electrodes (33, 34) separated from each other and connected to a high voltage energy generating circuit, and the means for focusing these shock waves comprising a semi-ellipsoidal reflector (17), the extremities (33a, 34a) of these electrodes being located essentially at one (Fₑ) of the focal points of the reflector (17), the other focal point (Fᵣ) of the reflector being centered on said lithiasis, said electrodes being equipped with means of wear compensation, said means comprising at least one compensation mechanism designed to axially displace the two electrodes, independently of each other, **characterized in that** said compensation mechanism is designed to displace these two electrodes along an axis corresponding to the axis of symmetry of the reflector (17) and to turn at least one of said electrodes on its longitudinal axis.

2. An apparatus according to claim 1, **characterized in that** one (33) of the electrodes is located along the axis of the reflector (17) and housed inside a tube (41) which is joined to a first mechanism designed to axially displace said tube with the electrode it contains and to cause said tube to rotate along its longitudinal axis.

3. An apparatus according to claim 1, **characterized in that** the other electrode (34) comprises two portions (44, 45) connected to each other by a connecting strip (46), one (44) of said portions being located along the axis of the reflector (17) and the other portion (45) being housed inside a tube (47) which is connected to a second mechanism designed to axially displace said tube with the electrode it contains.

4. An apparatus according to claim 1, **characterized in that** it comprises a computer designed to determine the spatial coordinates of the lithiasis (35) to be treated and of the reflector (17), means for emitting control signals as a function of the values determined for said spatial coordinates, and means for displacing said reflector as a function of said signals so as to position the receptor focal point (Fᵣ) on said lithiasis.

5. An apparatus according to claim 4, **characterized in that** the means for displacing the reflector (17) comprises a housing (40) that holds the vat constituting the reflector, a carriage to which the housing is attached, slides that are substantially horizontal and crossed allowing this carriage to be displaced in two orthogonal directions, slides that are substantially vertical allowing it to be displaced in a third direction perpendicular to the two other directions, as well as systems for activating this carriage according to these three directions.

6. An apparatus according to claim 1, **characterized in that** the live viewing device (60) includes a radioscopic equipment comprising an X-ray generator (62) and receiver means (63) of said X-rays, respectively attached to the two extremities of an arc-shaped arm (61) pivoting in its plane about its center, a display (18) for displaying the radioscopic images, and means for communicating the visual data to said computer in order to determine the relative coordinates of the emitting focal point (Fₑ) and of the lithiasis.

7. An apparatus according to claim 1, **characterized in that** the live viewing device (60) comprises an echography device as well as at least two cameras which are offset from each other, a display for displaying the echography images and means for communicating the visual data to said computer in order to determine the relative coordinates of the emitting focal point (Fₑ) and of the lithiasis.

8. An apparatus according to claim 7, **characterized in that** one of the cameras is located at the base of the apparatus, below the treatment zone, in order to furnish an image substantially perpendicular to this zone, and the other camera is offset to the side in order to furnish an image according to an oblique direction of this zone.
